Europäisches Patentamt

European Patent Office

Office européen des brevets

(11) Veröffentlichungsnummer : **0 128 497**
**B1**

(12) EUROPÄISCHE PATENTSCHRIFT

(45) Veröffentlichungstag der Patentschrift :
15.03.89

(51) Int. Cl.⁴ : **G 01 N 21/64**

(21) Anmeldenummer : 84106346.4

(22) Anmeldetag : 04.06.84

(54) Lumineszierende Schichten zur Verwendung in Vorrichtungen zur Bestimmung der Sauerstoffkonzentration in Gasen und dgl. durch Messung der Lumineszensverringerung.

(30) Priorität : 09.06.83 DE 3320752

(43) Veröffentlichungstag der Anmeldung :
19.12.84 Patentblatt 84/51

(45) Bekanntmachung des Hinweises auf die Patenterteilung : 15.03.89 Patentblatt 89/11

(84) Benannte Vertragsstaaten :
DE GB SE

(56) Entgegenhaltungen :
EP-A- 0 109 958
EP-A- 0 109 959
DE-A- 3 148 830
DE-B- 2 823 318

(73) Patentinhaber : Barnikol, Wolfgang, Prof. Dr.Dr.
Lanzelhohl 66
D-6500 Mainz-Bretzenheim (DE)

Burkhard, Oswald, Dr.
Schillerstrasse 9
D-6761 Kriegsfeld (DE)

(72) Erfinder : Barnikol, Wolfgang, Prof. Dr.Dr.
Lanzelhohl 66
D-6500 Mainz-Bretzenheim (DE)
Erfinder : Burkhard, Oswald, Dr.
Schillerstrasse 9
D-6761 Kriegsfeld (DE)

(74) Vertreter : Wolff, Hans Joachim, Dr.jur. Dipl.-Chem. et
al
Beil, Wolff & Beil Rechtsanwälte Postfach 80 01 40
Adelonstrasse 58
D-6230 Frankfurt am Main 80 (DE)

## Beschreibung

Die Erfindung betrifft den Gegenstand der Patentansprüche.

In der europäischen Patentanmeldung EP-A-83 703 (Priorität 10.12.81, Veröffentlichungsdatum 20.7.83) ist eine Vorrichtung zur Bestimmung der Sauerstoffkonzentration in Gasen, Flüssigkeiten und Geweben durch Messung der Verringerung der Lichtemission einer mit einem Anregungslicht bestimmter Wellenlänge bestrahlten lumineszierende Oberfläche vorgeschlagen worden, bei der diese Oberfläche durch einlagig dichtgepackte Teilchen mit einem Durchmesser von weniger als 1 mm, vorzugsweise weniger als 0,1 mm gebildet wird. Diese Oberfläche besteht entweder aus der lumineszierenden Substanz selbst oder die lumineszierende Substanz ist auf einem inerten Trägermaterial aufgezogen. In einer weiteren Ausbildung wird das Messgut von der Meßschicht durch eine sauerstoffdurchlässige Membran getrennt.

In der EP-A-109 959 (Priorität 23.11.82, Veröffentlichungsdatum 30.05.84) ist eine lumineszierende Schicht zur Bestimmung der Sauerstoffkonzentration durch Messung der Lichtemission dieser mit Anregungslicht bestrahlten Schicht vorgeschlagen worden, wobei die Schicht aus einer homogenen Mischung aus Lumineszenzfarbstoff und hydrophobem Silikonkautschuk besteht.

Es wurde nun gefunden, dass besonders gute und brauchbare Messergebnisse erzielt werden können, wenn die aus der Mischung aus Lumineszenzfarbstoff und licht- und sauerstoffdurchlässigem Material bestehende Schicht auf einen Träger mit feinkörniger und deshalb sehr grosser Oberfläche aufgebracht ist.

Dabei vereinfacht sich gegenüber den aus EP-A-83 703 bekannten Schichten die Herstellung erheblich, was insbesondere für eine industrielle Fertigung von Vorteil ist.

Die Eigenschaften des Schichtmaterials, das in allen Fällen gut licht- und sauerstoffdurchlässig sein muss, können je nach dem vorgesehenen Verwendungszweck ausgewählt werden. Damit die Mess-Signale vom Feuchtigkeitsgehalt der Probe unabhängig sind, benutzt man vorzugsweise eine hydrophobe Substanz als Schichtmaterial.

Zur Herstellung der erfindungsgemässen lumineszierenden Schichten können unter anderem die folgenden Methoden oder ihre Kombinationen benutzt werden :

1. Das Schichtmaterial und der Farbstoff werden gemeinsam in einem geeigneten Lösungsmittel oder einer Lösungsmittelkombination gelöst und die Lösung wird dann auf den Träger verteilt. Man erhält die lumineszierende Schicht nach Verdampfen des Lösungsmittels.

2. Der Farbstoff wird in das Schichtmaterial eingeschmolzen. Dazu werden das Schichtmaterial und der Farbstoff über den Schmelzpunkt erhitzt und die Schmelze auf dem Träger dünn verteilt. Nach dem Erkalten bildet sich die gewünschte lumineszierende Schicht.

3. Man mischt Monomere oder Oligomere und den Farbstoff, gegebenenfalls unter Zusatz eines geeigneten Lösungsmittels, verteilt die Mischung auf dem Träger und leitet die Polymerisation ein.

Als besonders geeignetes Schichtmaterial hat sich Silikonkautschuk erwiesen.

Da eine möglichst grosse Oberfläche der lumineszierenden Schicht wesentlich ist, wird die lumineszierende Schicht nicht auf einem planen Träger, wie beispielsweise einer Glasplatte, aufgebracht, sondern auf einem Träger, dessen Oberfläche eine feinkörnige Oberfläche aufweist. In Betracht kommt beispielsweise eine mit feinkörnigem Material, wie etwa Kieselgel, beschichtete Platte. Die Korngrösse des feinkörnigen Materials sollte kleiner als 1 mm, vorzugsweise kleiner als 0,1 mm sein.

Es wurde ferner gefunden, dass die Dicke der den lumineszierenden Farbstoff enthaltenden Schicht auf das Messergebnis keinen Einfluss hat, so dass bei der erfindungsgemässen Schicht durch Fertigungstoleranzen bedingte unterschiedliche Schichtdicken nicht nachteilig sind.

Beispiel

Eine 40 %ige Lösung von Silikonkautschuk in Toluol wurde im Verhältnis 1 : 3 mit Toluol verdünnt und zu 1,4 ml dieser Lösung 30 µl einer Lösung von 10 mg Fluorescent Yellow in 1 ml Toluol gegeben. 25 µl dieser Lösung wurden dann auf einem mit Kieselgel beschichteten Glasplättchen von ca. 1 cm Durchmesser verteilt.

Mit einer derartigen Schicht wurden in der Vorrichtung nach EP-A-83 703 90 %-Einstellzeiten von etwa 60 ms erreicht. Die $O_2$-Empfindlichkeit war zufriedenstellend und das Signal war nicht vom Feuchtigkeitsgehalt der Probe abhängig.

## Patentansprüche

1. Lumineszierende, auf einen Träger aufgebrachte Schicht zur Verwendung in Vorrichtungen zur Bestimmung der Sauerstoffkonzentration in Gasen, Flüssigkeiten und Geweben durch Messung der Verringerung der Lichtemission einer mit einem Anregungslicht bestimmter Wellenlänge bestrahlten lumineszierenden Oberfläche, bestehend aus der homogenen Mischung eines Lumineszenzfarbstoffes mit einem licht- und sauerstoffdurchlässigen sowie wasserabweisenden oder -abstossenden Schichtmaterial, wobei der Träger eine feinkörnige Oberfläche aufweist.

2. Lumineszierende Schicht nach Anspruch 1, dadurch gekennzeichnet, dass die Korngrösse der feinkörnigen Oberfläche kleiner als 1 mm, vorzugsweise kleiner als 0,1 mm ist.

3. Lumineszierende Schicht nach Anspruch 1 oder 2, dadurch gekennzeichnet, dass als Schichtmaterial Silikonkautschuk verwendet wird.

## Claims

1. Luminescent layer, laid on a carrier, for using in devices for measuring the oxygen concentration of gases, liquids and fabrics by measuring the diminution of the light emission of a luminescent surface under illumination from stimulating light of a certain wavelength, the surface comprising a homogeneous mixture of a luminescent dye with a layer material which is translucent and permeable to oxygen and is hydrophobic or water repellent, the carrier having a fine-grained surface.

2. Luminescent layer according to Claim 1, characterized in that the grain size of the fine-grained surface is less than 1 mm, preferably less than 0.1 mm.

3. Luminescent layer according to Claim 1 or Claim 2, characterized in that silicone rubber is used as the layer material.

## Revendications

1. Couche luminescente appliquée sur un support, pour être utilisée dans des dispositifs de détermination de la concentration en oxygène dans des gaz, liquides et tissus par mesure de la diminution de l'émission lumineuse d'une surface luminescente irradiée avec une lumière d'excitation, de longueur d'onde prédéterminée, composée du mélange homogène d'un pigment coloré luminescent avec un matériau en couche perméable à la lumière et à l'oxygène ainsi qu'hydrofuge ou hydrophobe, dans laquelle ledit support présente une surface à grain fin.

2. Couche luminescente selon la revendication 1, caractérisée en ce que la dimension des grains fins de la surface est inférieure à 1 mm, de préférence inférieure à 0,1 mm.

3. Couche luminescente selon l'une des revendications 1 ou 2, caractérisée en ce que du caoutchouc au silicone est utilisé comme matériau de couche.